# EUROPEAN PATENT APPLICATION

(11) **EP 3 991 561 A1**
(43) Date of publication of application: **04.05.2022**
(21) Application number: 21156513.0
(22) Date of filing: 11.02.2021
(51) Int. Cl.: A01N 43/16, A01N 65/03, A01P 1/00, A61K 8/9717, A61K 9/10, A61K 31/045, A61K 31/731, A61P 31/04, A61P 31/12

(54) **ANTIMICROBIAL DISINFECTANT GEL**

(30) Priority: 28.10.2020 DK PA202001213
(71) Applicant: Vepidan ApS, 9670 Loegstoer (DK)
(72) Inventor: IBSEN, Lars S., 9670 Loegstoer (DK)
(74) Representative: Sun, Yiming

(57) **Abstract**

An antimicrobial and antiviral disinfectant gel for hands is disclosed. The disinfectant gel comprises one or more polysaccharides such as iota- and kappa-carrageenan for the manufacture of an antiviral pharmaceutical composition for the treatment of a pathological condition or disease caused by or associated with an infection by a respiratory virus. The disinfectant gel comprises one or more ingredients includes iota-carrageenan, kappa-carrageenan, and purified water. The disinfectant gel counteracts infection from bacteria and virus particles. The disinfectant gel acts directly on bacteria and viruses by forming a special, physical/chemical binding to the surface proteins. The film acts as a shield against bacteria and viruses and at the same time encapsulates virus particles on the surface of the mucous membranes and skin. The disinfectant gel treats infections include tingling in the skin and throat, sore throat, sneezing, runny nose, fatigue, or soreness in bodies.

## Description

### TECHNICAL FIELD OF THE INVENTION

The invention disclosed herein generally relates to disinfectant gel. More particularly, the present invention relates to an antimicrobial disinfectant gel with polysaccharides and mixtures or combinations of various carrageenans to provide a prolonged antimicrobial effect and inhibit or reduce the possibility of transmission of viruses.

### BACKGROUND

Ionic polysaccharides have been found to possess antiviral activity against different viruses. The potential antiviral activity of polysaccharides extracted from, but not limited to, carrageenan afforded protection viruses. Carrageenan is an additive used to thicken, emulsify, and preserve foods and drinks. It's a natural ingredient that comes from red seaweed (also called Irish moss). Carrageenan is a polysaccharide obtained from the red algae. It is a structural component of seaweed and extracted from three main types, namely iota, kappa, and lambda, although there are other types as well, including kappa-II, mu, and nu carrageenan. Carrageenan has been used extensively in the food, pharmaceutical, and cosmetics industries as thickeners, gelling agents, and stabilizing and dispersing agents.

Extensive pharmacological and toxicological studies have been conducted. At 5 micrograms/ml, carrageenan prevents the cell monolayer from destruction by herpes simplex virus type 1 (HSV-1) growth. At 10 micrograms/ml, carrageenan reduces the formation of new infectious HSV-1 by almost five logs. When 10 micrograms/ml, carrageenan was added at the beginning of HSV-1 infection of HeLa cells, there was potent inhibition of viral protein synthesis, and the cells continued synthesis of cellular proteins. This has not occurred if carrageenan was added 1 h after HSV-1 infection. The use of methionine-labeled virions to analyze the entry of HSV-1 or semliki forest virions into cells indicated that carrageenan did not affect virus attachment or virus entry. Moreover, carrageenan did not block the early permeabilization of cells to the toxic protein alpha-sarcin. These results suggest that this sulfated-polysaccharide inhibits a step in virus replication subsequent to viral internalization but before the onset of late viral protein synthesis. Further, carrageenan is non-toxic by oral, dermal, and inhalation routes of administrations even at extremely high doses.

Currently, antiviral compositions derived from different materials have been used for a long time and whose safety has been confirmed, especially antiviral composition useful as active ingredients for pharmaceuticals or functional foods are required. However, a satisfactory composition provided with polysaccharides and mixtures or combinations of various carrageenans has not yet been developed to effectively possess antiviral activity against different viruses.

Therefore, there is a need for a composition provided with polysaccharides and mixtures or combinations of various carrageenans to possess specific physical and chemical properties when they are formulated to provide a prolonged antimicrobial effect and inhibit or reduce the possibility of transmission of viruses.

### SUMMARY OF THE INVENTION

This summary is provided to introduce a selection of concepts in a simplified form that is further disclosed in the detailed description of the invention. This summary is not intended to identify key or essential inventive concepts of the claimed subject matter, nor is it intended for determining the scope of the claimed subject matter.

The present invention discloses disinfectant gel. More particularly, the present invention relates to an antimicrobial disinfectant gel with polysaccharides and mixtures or combinations of various carrageenans to provide a prolonged antimicrobial effect and inhibit or reduce the possibility of transmission of viruses.

In one embodiment, the disinfectant gel fights colds and flu-like illnesses. In one embodiment, the disinfectant gel acts directly on the bacteria and viruses by forming a special, physical/chemical binding to the surface proteins. In one embodiment, the film acts as a shield against bacteria and viruses. At the same time, the film encapsulates microbes such as bacteria and virus particles on the surface of the mucous membranes and skin.

In one embodiment, the disinfectant gel is an antimicrobial and antiviral disinfectant composition. In one embodiment, the disinfectant gel comprises one or more ingredients includes carrageenan and purified water. In one embodiment, the carrageenan could be iota-carrageenan (Carragelose^{®}) and/or kappa-carrageenan. In an exemplary embodiment, the carrageenan could be genuvisco (GENUYISCO^{®}) CI-123. In one embodiment, the purified water could be dest distilled water. In an exemplary embodiment, 1 ml of disinfectant gel contains about 1 to about 50 mg of carrageenan and purified water. In one embodiment, the disinfectant gel further comprises iso-propyl alcohol (IPA spirit 96%), lemon oil, and capryhen. The disinfectant gel counteracts infections from microbes such as bacteria and virus particles. The disinfectant gel encapsulates the microbes, for example, bacteria and virus particles on the surface of the mucous membranes and skin, thereby avoiding and/or reducing the multiplication and spread of infectious matters.

In one embodiment, a method of preparing the disinfectant gel comprises the following steps. At first step, one or more ingredients include IPA spirit 96%, genuvisco CI-123, and lemon oil are weighed and the powder is stirred into ethanol to a uniform slurry. The ingredients are weighed using a weight measuring unit and stirred inside a mixing unit using a stick blender. In one embodiment, the mixing unit is a 3-liter plastic bucket. At second step, the purified water is weighed and mixed to the uniform slurry obtained from the first step. The mixture is continuously stirred with constant speed to form a homogeneous solution. At another step, capryhen is weighed and mixed to the solution obtained from the second step to form a final homogeneous solution.

In one embodiment, the disinfectant gel is used at the first sign of a cold at least three times a day with one breath in each nostril. In one embodiment, the disinfectant gel comprises one or more polysaccharides such as iota- and/or kappa-carrageenan for the manufacture of an antiviral pharmaceutical composition for the treatment of a pathological condition or disease caused by or associated with an infection by a respiratory virus. In one embodiment, the nasal spray head is inserted carefully into the nose to encapsulate microbes such as bacteria and virus particles on the surface of the mucous membranes and skin. The disinfectant gel is sprayed at least three times a day with one puff in each nostril. The viruseptin nasal spray container is held vertically when spraying the disinfectant gel.

In one embodiment, the disinfectant gel is a clear viscous solution without any odor. In one embodiment, the disinfectant gel treats infections include tingling in the skin and throat, sore throat, sneezing, runny nose, fatigue, or soreness in bodies. In one embodiment, the disinfectant gel is provided in at least any one or more forms including a spray, liquid, lotion, cream, and wet wipes.

In one embodiment, a method of treating infection from bacteria and virus particles, comprising administrating a disinfectant gel comprising one or more ingredients includes iota-carrageenan (Carragelose^{®}), kappa-carrageenan, and purified water. In one embodiment, the disinfectant gel is sprayed at the first signs of a cold. In one embodiment, the disinfectant gel is used to treat infections include, but not limited to, tingling in the skin and throat, sore throat, sneezing, runny nose, fatigue, or soreness in bodies. The treatment is started as early as possible after the first symptoms and continued until the symptoms subside.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### DETAILED DESCRIPTION OF THE INVENTION

According to the present invention, a disinfectant gel or disinfecting composition is prepared for the prevention of microbes such as bacteria and viruses from hands. In one embodiment, the disinfectant gel fights colds and flu-like illnesses. In one embodiment, the disinfectant gel acts directly on the bacteria and viruses by forming a special, physical/chemical binding to the surface proteins. In one embodiment, the film acts as a shield against bacteria and virus. At the same time, the film encapsulates microbes such as bacteria and virus particles on the surface of the mucous membranes and skin.

In one embodiment, the disinfectant gel is an antimicrobial and antiviral disinfectant composition. In one embodiment, the disinfectant gel comprises one or more ingredients includes carrageenan and purified water. In one embodiment, the carrageenan could be iota-carrageenan (Carragelose^{®}) and/or kappa-carrageenan. In an exemplary embodiment, the carrageenan could be genuvisco (GENUYISCO^{®}) CI-123. In one embodiment, the purified water could be dest distilled water. In an exemplary embodiment, 1 ml of disinfectant gel contains about 1 to about 50 mg of carrageenan and purified water. In one embodiment, the disinfectant gel further comprises iso-propyl alcohol (IPA spirit 96%), lemon oil, and capryhen. The disinfectant gel counteracts infections from microbes such as bacteria and virus particles. The disinfectant gel encapsulates the microbes, for example, bacteria and virus particles on the surface of the mucous membranes and skin, thereby avoiding and/or reducing the multiplication and spread of infectious matters.

In one embodiment, a method of preparing the disinfectant gel comprises the following steps. At first step, one or more ingredients include IPA spirit 96%, genuvisco CI-123, and lemon oil are weighed and the powder is stirred into ethanol to a uniform slurry. The ingredients are weighed using a weight measuring unit and stirred inside a mixing unit using a stick blender. In one embodiment, the mixing unit is a 3 liter plastic bucket. In one embodiment, the uniform slurry has IPA spirit 96% of about 50 gr. In one embodiment, the uniform slurry has genuvisco of about 40 gr. In one embodiment, the uniform slurry has lemon oil of about 5.33 gr. At second step, the purified water is weighed and mixed with the uniform slurry obtained from the first step. In one embodiment, the purified water of about 1,884.67 gr is mixed with the uniform slurry. The mixture is continuously stirred with constant speed to form a homogeneous solution. At another step, capryhen is weighed and mixed to the solution obtained from the second step to form a final homogeneous solution. In one embodiment, the capryhen of about 20 gr is weighed and mixed to the solution obtained from the second step.

In one embodiment, the disinfectant gel is used at the first sign of a cold at least three times a day with one breath in each nostril. In one embodiment, the disinfectant gel is stored in a viruseptin nasal spray container. The container has a container body, a nasal spray head, and a protective cap. The disinfectant gel is stored in the container body. During use, the protective cap is removed and the nasal spray head is pumped one or more times. In one embodiment, the nasal spray head is pumped a few times until a uniform atomized spray is obtained. The nasal spray head is closed using the protective cap after use.

In one embodiment, the disinfectant gel comprises one or more polysaccharides such as iota- and/or kappa-carrageenan for the manufacture of an antiviral pharmaceutical composition for the treatment of a pathological condition or disease caused by or associated with an infection by a respiratory virus. In one embodiment, the nasal spray head is inserted carefully into the nose to encapsulate microbes such as bacteria and virus particles on the surface of the mucous membranes and skin. The disinfectant gel is sprayed at least three times a day with one puff in each nostril. The viruseptin nasal spray container is held vertically when spraying the disinfectant gel.

In one embodiment, the container stores about 20 ml of disinfectant gel. In one embodiment, the disinfectant gel contains lemon oil. The lemon oil provides a pleasant scent for a period of use. Further, the container is categorized as a CE marked medical device. The container is stored in a temperature ranges from about 5 degrees to about 30 degrees. The container should be protected from light and out of reach of children.

In one embodiment, a method of using the disinfectant gel comprises the following steps. At one step, the hands are thoroughly washed with hand-wash and water, thereby removing dirt and impurities from hands. The hand-wash could be in the form of hand soap or liquid. The water could be hot or cold as needed. The washed hand is wiped thoroughly with clean paper or a towel. At another step, the hands are disinfected with hand alcohol disinfectant gel. In one embodiment, one or more pump strokes of disinfectant gel is sprayed in one hand. The gel is thoroughly distributed over both hands, fingers, and spaces, as well as the back of the hand and wrist. The lubrication of the gel is continued until the gel begins to dry up. The hands move calmly through the air sometimes until both hands are completely dry.

At another step, the hands are lubricated with protective gel. In one embodiment, one or more pump strokes of protective gel is sprayed in one hand. The gel is thoroughly distributed over both hands, fingers, and spaces, as well as the back of the hand and wrist. The lubrication of the gel is continued for about 30 seconds until the gel begins to dry up. The hands move calmly through the air sometimes until both hands are completely dry. In one embodiment, the hands are cleaned and the treatment lasts for about 3-4 hours. The treatment could be repeated as needed.

In one embodiment, the disinfectant gel is a clear viscous solution without odor. In one embodiment, the disinfectant gel treats infections include tingling in the skin and throat, sore throat, sneezing, runny nose, fatigue, or soreness in bodies. In one embodiment, the disinfectant gel is provided in at least any one or more forms including a spray, liquid, lotion, cream, and wet wipes.

In one embodiment, a method of treating infection from bacteria and virus particles, comprising administrating a disinfectant gel comprising one or more ingredients includes iota-carrageenan (Carragelose^{®}), kappa-carrageenan, and purified water. In one embodiment, the disinfectant gel is sprayed at the first signs of a cold. In one embodiment, the disinfectant gel is used to treat infections include, but not limited to, tingling in the skin and throat, sore throat, sneezing, runny nose, fatigue, or soreness in bodies. The treatment is started as early as possible after the first symptoms and continued until the symptoms subside.

The disinfectant gel comprises one or more polysaccharides and mixtures or combinations of various carrageenans to possess specific physical and chemical properties when they are formulated, thereby providing a prolonged antimicrobial effect and inhibiting or reducing the possibility of transmission of viruses. The disinfectant gel could be used by adults and children of 1 year. The disinfectant gel could also be used by pregnant and lactating women.

The foregoing examples have been provided merely for the purpose of explanation and are in no way to be construed as limiting of the present concept disclosed herein. While the concept has been described with reference to various embodiments, it is understood that the words, which have been used herein, are words of description and illustration, rather than words of limitation. Further, although the concept has been described herein with reference to particular means, materials, and embodiments, the concept is not intended to be limited to the particulars disclosed herein; rather, the concept extends to all functionally equivalent structures, methods and uses, such as are within the scope of the appended claims. Those skilled in the art, having the benefit of the teachings of this specification, may affect numerous modifications thereto and changes may be made without departing from the scope and spirit of the concept in its aspects.

## Claims

1. An antimicrobial and antiviral disinfectant gel for hands, comprising:
one or more ingredients include iota-carrageenan (Carragelose^{®}), kappa-carrageenan, and purified water.

2. The disinfectant gel of claim 1, wherein 1ml of disinfectant gel contains about 1 to about 50 mg of carrageenan and purified water.

3. The disinfectant gel of claim 1, further comprises iso-propyl alcohol, lemon oil, and capryhen.

4. The disinfectant gel of claim 1, counteracts infection from bacteria and virus particles.

5. The disinfectant gel of claim 1, acts directly on bacteria and viruses by forming a special, physical/chemical binding to the surface proteins.

6. The disinfectant gel of claim 5, wherein the film acts as a shield against bacteria and viruses and at the same time encapsulates virus particles on the surface of the mucous membranes and skin.

7. The disinfectant gel of claim 1, is a clear viscous solution without odor.

8. The disinfectant gel of claim 1, treats infections include tingling in the skin and throat, sore throat, sneezing, runny nose, fatigue, or soreness in bodies.

9. The disinfectant gel of claim 1, is provided in at least any one or more forms including a spray, liquid, lotion, cream, and wet wipes.
